(19) Europäisches Patentamt
European Patent Office
Office européen des brevets

(11) Veröffentlichungsnummer: **0 195 361**
**A2**

(12) # EUROPÄISCHE PATENTANMELDUNG

(21) Anmeldenummer: 86103337.1

(51) Int. Cl.[4]: A 61 K 7/13

(22) Anmeldetag: 12.03.86

(30) Priorität: 20.03.85 DE 3510039

(43) Veröffentlichungstag der Anmeldung: 24.09.86 Patentblatt 86/39

(84) Benannte Vertragsstaaten: AT BE CH DE FR GB IT LI LU NL SE

(71) Anmelder: Henkel Kommanditgesellschaft auf Aktien
Postfach 1100 Henkelstrasse 67
D-4000 Düsseldorf-Holthausen(DE)

(72) Erfinder: Rose, David, Dr.
Holbeinweg 7
D-4010 Hilden(DE)

(72) Erfinder: Maak, Norbert, Dr.
Im Jagdfeld 41 a
D-4040 Neuss(DE)

(72) Erfinder: Lieske, Edgar
Hunsrückenstrasse 40
D-4000 Düsseldorf(DE)

(54) **Haarfärbemittel.**

(57) Oxidationshaarfärbemittel, die als Kupplersubstanzen Aminodiphenylamine der allgemeinen Formel I

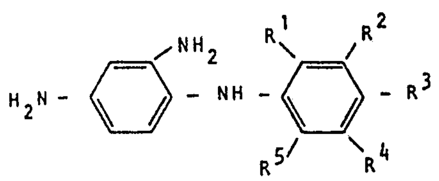

in der eine der Gruppen $R^1$-$R^5$ eine $-SO_3H$, $-COOH$ oder $-CH_2-COOH$ Gruppe und die übrigen Wasserstoff, eine Alkylgruppe mit 1 bis 4 C-Atomen, eine Alkoxygruppe mit 1 bis 4 C-Atomen oder ein Chloratom sein können, wobei wenigstens zwei der Gruppen $R^1$-$R^5$ Wasserstoff darstellen oder deren Salze und die in Oxidationsfärbemitteln üblichen Entwicklersubstanzen enthalten, ergeben intensive Haaranfärbungen mit hoher Echtheit. Mit Entwicklersubstanzen vom Typ der aromatischen Diamine werden besonders schöne braune Nuancen erhalten. Bevorzugt stellt eine der Gruppen $R^1$-$R^5$ eine $SO_3H$ oder $-COOH$-Gruppe dar, von den übrigen sind wenigstens 3 Wasserstoff und gegebenenfalls die übrigen Methylgruppen.

Henkel KGaA
ZR-FE/Patente

1

Henkelstraße 67
4000 Düsseldorf, den 15.3.1985

Dr. JG/Po

## P a t e n t a n m e l d u n g

D 7277 EP

## "H a a r f ä r b e m i t t e l"

Gegenstand der Erfindung sind Haarfärbemittel auf der Basis von Oxidationsfarbstoffen. Solche Haarfärbemittel enthalten Oxidationsfarbstoffvorprodukte in einem kosmetischen Träger. Als Oxidationsfarbstoffvorprodukte werden Entwicklersubstanzen und Kupplersubstanzen eingesetzt, die unter dme Einfluß von Oxidationsmitteln oder von Luftsauerstoff Farbstoffe ausbilden.
Als kosmetische Träger für die Oxidationsfarbstoffvorprodukte dienen Cremes, Emulsionen, Gele, Shampoos, Schaumaerosole oder andere Zubereitungen, die für die Anwendung auf dem Haar geeignet sind.

Für das Färben von Haaren spielen die sogenannten Oxidationsfarben, die durch oxidative Kupplung einer oder mehrerer Entwicklerkomponenten untereinander oder mit einer oder mehreren Kupplerkomponenten entstehen, wegen ihrer intensiven Farben und guten Echtheitseigenschaften eine bevorzugte Rolle. Als Entwicklersubstanzen werden üblicherweise primäre aromatische Amine mit einer weiteren in Para- oder Orthoposition befindlichen freien oder substituierten Hydroxy- oder Aminogruppe, ferner Diaminopyridinderivate, heterocyclische Hydrazonderivate, 4-Aminopyrazolonderivate und Tetraaminopyrimidine eingesetzt. Als sogenannte Kupplersubstanzen werden m-Phenylendiaminderivate, Naphthole, Resorcinderivate und Pyrazolone verwendet.

...

Gute Oxidationshaarfarbstoffvorprodukte müssen in erster Linie folgende Voraussetzungen erfüllen: Sie müssen bei der oxidativen Kupplung die gewünschte Farbnuancen in ausreichender Intensität ausbilden. Sie müssen ferner ein gutes Aufziehvermögen auf menschlichem Haar besitzen, ohne die Kopfhaut zu stark anzufärben. Die damit erzeugten Färbungen müssen eine hohe Stabilität gegen Wärme, Licht und die bei der Dauerwellung des Haars verwendeten Chemikalien aufweisen. Schließlich sollen die Oxidationshaarfarbstoffvorprodukte in toxikologischer und dermatologischer Hinsicht unbedenklich sein.

Es wurde gefunden, daß Haarfärbemittel, enthaltend Oxidationsfarbstoffvorprodukte in einem Träger, die als Oxidationsfarbstoffvorprodukte Aminodiphenylamine der Formel I

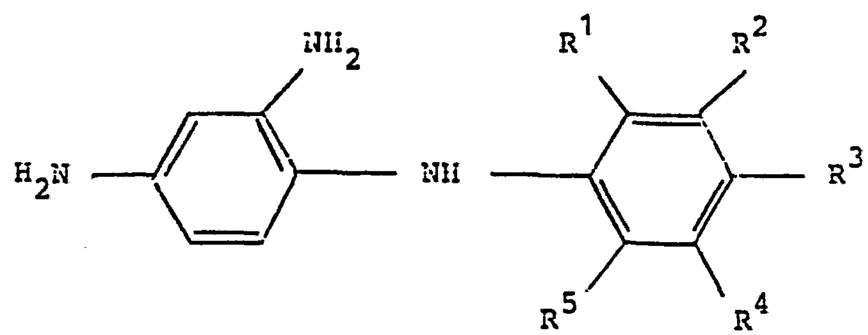

(I)

in der eine der Gruppen $R^1$ - $R^5$ eine $-SO_3H$, -COOH oder eine $-CH_2COOH$-Gruppe und die übrigen Wasserstoff, eine Alkylgruppe mit 1 - 4 C-Atomen, eine Alkoxygruppe mit 1 - 4 C-Atomen oder ein Chloratom sein können, wobei wenigstens zwei der Gruppen $R^1$ - $R^5$ Wasserstoff darstellen,, oder deren Salze als Kupplersubstanzen und die in Oxidationshaarfärbemitteln üblichen Entwicklersubstanzen enthalten, die gestellten Anforderungen in hohem Maße erfüllen.

Die Aminodiphenylamine der allgemeinen Formel I sind als Kupplersubstanzen für eine Vielzahl bekannter Entwicklerverbindungen geeignet und erzeugen besonders brillante Färbungen mit

...

hoher Licht- und Wärmestabilität und hoher Stabilität gegen Kaltwellbehandlung. Hervorzuheben sind die besonders schönen, der natürlichen Haarfarbe nahekommenden Brauntöne, die mit Entwicklern vom Typ der aromatischen Diamine erhalten werden.

Bevorzugt geeignete zur Herstellung erfindungsgemäßer Haarfärbemittel sind Aminodiphenylamine der Formel I, in der eine der Gruppen $R^1$ - $R^5$ eine $-SO_3H$-Gruppe oder -COOH-Gruppe und die übrigen Wasserstoff oder Methylgruppen sind, wobei wenigstens 3 dieser Gruppen Wasserstoff sind.

Verbindungen der allgemeinen Formel I sind teilweise literaturbekannt. Die nicht literaturbekannten Verbindungen dieser Struktur lassen sich nach ansich bekannte Verfahren herstellen. So ist z.B. 2.4-Diaminodiphenylamin-4'-sulfonsäure aus DE-PS 163 645 bekannt. Die Herstellung von 2.4-Diamino-4'-carboxydiphenylamin und 2.4-Diamino-3'-carboxydiphenylamin ist in Journ. Prakt. Chem. (2), 91 (1915), S. 202 - 212 beschrieben. Allgemein können Aminodiphenylamine der Formel I durch Umsetzung eines Aminobenzolderivats der Formel II

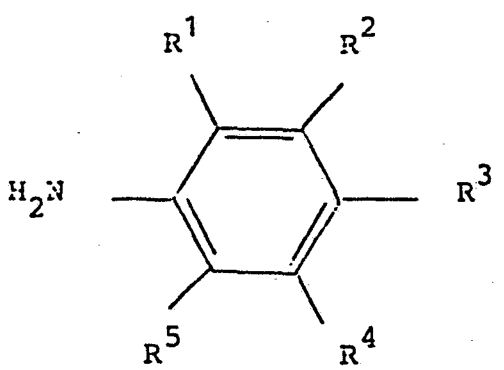

(II)

in der die Gruppen $R^1$ - $R^5$ die für die Formel I genannte Bedeutung haben, mit 2.4-Dinitrofluorbenzol und katalytische Hydrierung der dabei erhaltneen 2.4-Dinitrodiphenylamine zu den entsprechenden 2.4-Diaminodiphenylaminen hergestellt werden. Einige Beispiele für die Herstellung nicht literaturbekannter Aminodiphenylamine werden weiter unten beschrieben.

. . .

In die erfindungsgemäßen Haarfärbemittel können die Aminodiphenylamine der Formel I in freier Form (bzw. in Form ihrer inneren Salze) oder in Form von Salzen der Aminogruppen mit anorganischen oder organischen Säuren, z.B. in Form der Hydrochloride, Sulfate, Phosphate, Acetate, Propionate, Lactate oder Citrate oder in Form von Salzen der $-SO_3H$, -COOH und $-CH_2$-COOH-Gruppe, z.B. der Ammoniumsalze oder der Alkalisalze eingesetzt werden.

Als Entwicklersubstanzen können in den erfindungsgemäßen Haarfärbemitteln z.B. aromatische Amine mit einer oder mehreren weiteren $NH_2$-Gruppen, NHR-Gruppen, $NR_2$-Gruppen, wobei R eine Alkylgruppe mit 1 - 4 C-Atomen oder eine Hydroxyalkylgruppe oder eine Aminoalkylgruppe mit 2 - 4 C-Atomen darstellt, Aminophenole, Aminophenolether, Diaminopyridinderivate oder 2.4.5.6-Tetraaminopyrimidin und dessen Derivate wie z.B. 4.5-Diamino-2.6-bis-methylaminopyrimidin, 2.5-Diamino-4-diethylamino-6-methylaminopyrimidin, 2.4.5-Triamino-6-anilinopyrimidin, 2.4.5-Triamino-6-morpholino-pyrimidin, 2.4.5-Triamino-6-(2-hydroxyethyl)-aminopyrimidin.

Besonders intensive und natürliche braune bis graue Farbnuancen werden bei Verwendung der neuen Kupplersubstanzen in Oxidationsfärbemitteln zusammen mit Entwicklersubstanzen vom Typ der aromatischen Diamine, insbesondere der p-Phenylendiaminderivate erhalten.

Solche bevorzugten Entwicklersubstanzen sind z.B. p-Phenylendiamin, p-Toluylendiamin, N-Methyl-p-phenylendiamin, N,N-Dimethyl-p-phenylendiamin, N-Hydroxyethyl-p-phenylendiamin, N,N-Bis-(2-hydroxyethyl)-p-phenylendiamin, N-Ethyl-N-(2-hydroxyethyl)-p-phenylendiamin, N,N-Diethyl-2-methyl-p-phenylendiamin, 2-Chlor-p-phenylendiamin, 2,6-Dichlor-p-phenylendiamin, 2-Chlor-6-methyl-p-phenylendiamin, 2-Methoxy-p-phenylendiamin, 2.5-Diaminoanisol, 6-Methoxy-3-methyl-p-phenylendiamin, N-(2-

...

Methoxyethyl)-p-phenylendiamin, N-(2-Hydroxypropyl)-p-phenylendiamin, N-Butyl-N-sulfobutyl-p-phenylendiamin, N-(p-Aminophenyl)-N',N'-bis-(ß-hydroxyethyl)-1.3-diaminopropan oder deren Salze mit anorganischen oder organischen Säuren.

Die erfindungsgemäßen Haarfärbemittel können neben den Aminodiphenylaminen der allgemeinen Formel I auch andere bekannte Kupplersubstanzen enthalten, die zur Modifizierzung der Farbnuancen und zur Erzeugung natürlicher Farbtöne erforderlich sind. Solche üblichen Kupplerverbindungen sind z.B. andere m-Phenylendiamine, z.B. 2.4-Diaminophenyl-2-hydroxyethylether, 2.4-Diaminoanisol oder Phenole, Resorcine, m-Aminophenole, Naphthole oder Pyrazolone.

Gegebenenfalls können auch direktziehende Farbstoffe zusätzlich zur weiteren Modifizierung der Farbnuancen eingesetzt werden. Solche direktziehenden Farbstoffe sind z.B. Nitrophenylendiamine, Nitroaminophenole, Anthrachinonfarbstoffe oder Indophenole.

Zu den erfindungsgemäßen Haarfärbemitteln werden die Aminodiphenylaminde der Formel I und die gegebenenfalls zusätzlich vorhandenen bekannten Kupplersubstanzen im allgemeinen in etwa molaren Mengen, bezogen auf die verwendeten Entwicklersubstanzen, eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklersubstanzen und Kupplersubstanzen in einem Molverhältnis von 1 : 0,5 bis 1 : 2 enthalten sein können.

Es ist nicht erforderlich, daß die Aminodiphenylamine der Formel I sowie die sonst in den Haarfärbemitteln vorhandenen Oxidationsfarbstoffvorprodukte oder direkt ziehenden Farbstoffe einheitliche chemische Verbindungen darstellen. Vielmehr können diese auch Gemische der erfindungsgemäß einzusetzenden Kuppler- oder Entwicklersubstanzen sein.

...

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt am Haar gewünscht wird. Als Oxidationsmittel kommen insbesondere Wasserstoffperoxid oder dessen Anlagerungsprodukte an Harnstoff, Melamin oderNatriumborat sowie Gemische aus derartigen Wasserstoffperoxidanlagerungsprodukten mit Kaliumperoxiddisulfat in Betracht.

Zur Herstellung der erfindungsgemäßen Haarfärbemittel werden die Oxidationsfarbstoffvorprodukte in einen geeigneten kosmetischen Träger eingearbeitet. Solche Träger sind z.B. Cremes, Emulsionen, Gele oder auch tensidhaltige, schäumende Lösungen, z.B. Shampoos oder andere Zubereitungen, die für die Anwendung auf dem Haar geeignet sind. Übliche Bestandteile solcher kosmetischer Zubereitungen sind z.B. Netz- und Emulgiermittel wie anionische, nichtionische oder ampholytische Tenside, z.B. Fettalkoholsulfate, Alkansulfonate, alpha-Olefinsulfonate, Fettalkoholpolyglykolethersulfate, Ethylenoxidanlagerungsprodukte an Fettalkohole, Fettsäuren und Alkylphenole, Sorbitanfettsäureester und Fettsäurepartialglyceride, Fettsäurealkanolamide sowie Verdickungsmittel wie z.B. Methyl- oder Hydroxvethylcellulose, Stärke, Fettalkohole, Paraffinöle, Fettsäuren, ferner Parfümöle und haarpflegende Zusätze, wie z.B. wasserlösliche kationische Polymere, Proteinderivate, Pantothensäure und Cholesterin.

Die Bestandteile der kosmetischen Träger werden zur Herstellung der erfindungsgemäßen Haarfärbemittel in für diese Zwecke üblichen Mengen eingesetzt, z.B. werden Emulgiermittel in Konzentrationen von 0,5 - 30 Gew.-% und Verdickungsmittel in Konzentrationen von 0,1 - 25 Gew.-% des gesamten Färbemittels eingesetzt. Die Oxidationsfarbstoffvorprodukte werden in Mengen von 0,2 - 5 Gew.-%, vorzugsweise 1 - 3 Gew.-% des gesamten Färbemittels in den Träger eingemischt. Der Gehalt an Aminodiphenylaminen Formel I kann in den erfindungsgemäßen Haarfärbemitteln etwa 0,05 - 10 Millimol pro 100 g des Haarfärbemittels betragen.

. . .

Die Anwendung der erfindungsgemäßen Haarfärbemittel kann, unabhängig von der Art der kosmetischen Zubereitung, z.B. als Creme, Gel oder Shampoo im schwach sauren, neutralen oder alkalischen Milieu erfolgen. Bevorzugt ist die Anwendung der Haarfärbemittel in einem pH-Bereich von 8 - 10. Die Anwendungstemperaturen können in einem Bereich zwischen 15°C und 40°C liegen. Nach einer Einwirkungszeit von ca. 30 Minuten wird das Haarfärbemittel durch Ausspülen von dem zu färbenden Haar entfernt. Danach wird das Haar mit einem milden Shampoo nachgewaschen und getrocknet. Das Nachwaschen mit einem Shampoo entfällt, wenn ein stark tensidhaltiger Träger, z.B. ein Färbeshampoo verwendet wurde.

Die nachfolgenden Beispiele sollen den Erfindungsgegenstand näher erläutern ohne ihn jedoch hierauf zu beschränken.

...

## B e i s p i e l e

### 1. Herstellung von Aminodiphenylaminen der Formel I

#### 1.1) 2.4-Diaminodiphenylamin-2'-sulfonsäure-Na-Salz

1. Stufe

Eine Mischung aus 17,3 g (0,1 Mol) o-Sulfanilsäure, 4,0 g (0,1 Mol) Natriumhydroxid, 8,2 g (0,1 Mol) $CH_3COONa$, 12,6 g (0,1 Mol) und 2.4-Dinitrofluorbenzol in 100 ml Wasser wurde 3 Stunden unter Rückflußkühlung zum Sieden erhitzt. Nach dem Abkühlen wurde das Reaktionsprodukt abfiltriert und bei 80°C im Vakkum getrocknet. Es wurden 29,5 g (74 % d.Th.) des bei 274°C (unter Zersetzung) schmelzenden 2.4-Dinitrodiphenylamin-2'-sulfonsäure-Na-Salz erhalten.

2. Stufe

6,0 g 2.4-Dinitrodiphenylamin-2'-sulfonsäure-Na-Salz wurden in 250 ml Ethanol gelöst, mit 0,5 g einer mit 5 Gew.-% Palladium beladenen Aktivkohle versetzt und in Wasserstoffatmosphäre hydriert. Nach beendeter Wasserstoffaufnahme wurde der Katalysator abgetrennt und die Lösung zur Trockene eingeengt. Es wurden rosa Kristalle mit einem Schmelzpunkt oberhalb 225°C erhalten.

#### 1.2) 2.4-Diaminodiphenylamin-3'-sulfonsäure-Na-Salz

Nach dem unter 1.1 beschriebenen Verfahren wurde ausgehend von m-Sulfanilsäure das 2.4-Dinitrodiphenylamin-3'-sulfonsäure-Na-Salz in Form von orangefarbenen Kristallen mit einem Schmelzpunkt von 195°C (unter Zersetzung) erhalten. Durch katalytische Hydrierung analog 1.1 wurde daraus das 2.4-Diaminodiphenylamin-3'-sulfonsäure-Na-Salz in Form beiger Kristalle mit einem Schmelzpunkt ab 175°C (unter Zersetzung) erhalten.

. . .

1.3) 2.4-Diamino-4'-methyldiphenylamin-2'-sulfonsäure-Na-Salz

Nach dem unter 1.1 beschriebenen Verfahren wurde ausgehend von 6-Amino-m-toluolsulfonsäure das 2.4-Dinitro-4'-methyldiphenylamin-2'-sulfonsäure-Na-Salz in Form von orangefarbenen Kristallen mit einem Schmelzpunkt von oberhalb 250°C erhalten. Daraus wurde durch katalytische Hydrierung das 2.4-Diamino-4'-methyldiphenylamin-2'-sulfonsäure-Na-Salz in Form roter Kristalle mit einem Schmelzpunkt von über 250°C erhalten.

1.4) 2.4-Diaminodiphenylamin-4'-sulfonsäure, Na-Salz

1. Stufe

Eine Mischung aus 18,6 g (0,1 Mol) 2.4-Dinitrofluorbenzol, 17,3 g (0,1 Mol) p-Sulfanilsäure, 4 g (0,1 Mol) Natriumhydroxid und 8,2 g (0,1 Mol) Natriumacetat in 100 ml Wasser wurde 3 Stunden unter Rückfluß zum Sieden erhitzt. Nach Abkühlung auf 20°C wurde das Reaktionsprodukt abfiltriert und bei 80°C im Vakuum getrocknet. Das erhaltene 2.4-Dinitro-diphenylamin-4'-sulfonsäure-Na-Salz schmiltzt bei ca. 285°C (unter Zersetzung).

2. Stufe

6 g des 2.4-Dinitrodiphenylamin-4'-sulfonsäure-Na-Salz wurden in einer Mischung aus 250 ml Ethanol und 50 ml Wasser gelöst und nach Zugabe von 0,5 g einer mit 5 Gew.-% Palladium beladenen Aktivkohle in Wasserstoffatmosphäre hydriert. Nach beendeter Wasserstoff-Aufnahme wurde der Katalysator durch Filtration abgetrennt und das Filtrat zur Trockene eingeengt. Das 2.4-Diaminodiphenylamin-4'-sulfonsäure-Na-Salz wurde in Form rosafarbener Kristalle mit einem Schmelzpunkt oberhalb 310°C erhalten.

. . .

1.5) 2.4-Diamino-2'-carboxy-4'-methyldiphenylamin-trihydrochlrorid

1. Stufe

Eine Mischung aus 9,4 g (0,05 Mol) 2.4-Dinitrofluorbenzol, 7,7 g (0,05 Mol) 2-Amino-5-methylbenzoesäure, 6,0 g (0,005 Mol) $Na_2CO_3$ in 100 ml Ethanol-Wasser(1 : 1)-Gemisch wurde 3 Stunden bei 40°C gerührt. Nach Abkühlung auf + 10°C wurde das Reaktionsprodukt abfiltriert, mit Ethanol gewaschen und bei 60°C im Vakuum getrocknet. Es wurden 15,6 g (91 % d.Th.) roter Kristalle mit einem Schmelzpunkt oberhalb 250°C erhalten.

2.Stufe

Das 2.4-Dinitro-2'-carboxy-4'-methyldiphenylamin-Na-Salz (aus der 1. Stufe) wurde in 500 ml Ethanol gelöst und nach Zugabe von 1 g einer mit 5 Gew.-% Palladium beladenen Aktivkohle in Wasserstoffatmosphäre hydriert. Nach beendeter Wasserstoffaufnahme wurde der Katalysator durch Filtration abgetrennt und das Filtrat mit konzentrierter Salzsäure angesäuert. Nach dem Einengen wurden 10,2 g 2.4-Diamino-2'-carboxy-4'-methyldiphenylamin-trihydrochlorid in Form farbloser Kristalle mit einem Schmelzpunkt von 220° - 225°C (unter Zersetzung) erhalten.

1.6) 2.4-Diamino-3'-carboxy-6'-methyldiphenylamin-trihydrochlorid

Nach dem Verfahren gemäß 1.4 wurde ausgehend von 3-Amino-4-methyl-benzoesäure das 2.4-Dinitro-3'-carboxy-6'-methyldiphenylamin-Na-Salz in Form orangefarbener Kristalle mit einem Schmelzpunkt von 187°C (unter Zersetzung) erhalten. Daraus wurde durch katalytische Hydrierung das 2.4-Diamino-3'-carboxy-6'-methyldiphenylamin-tetrahydrochlorid in Form grauer Kristalle mit einem Schmelzpunkt von 249° C (unter Zersetzung) erhalten.

...

1.7) <u>2.4-Diamino-3'-carboxy-6'-methoxydiphenylamin-dihydrochlorid-dihydrat</u>

Nach dem Verfahren gemäß 1.4 wurde ausgehend von 3-Amino-4-methoxybenzoesäure das 2.4-Dinitro-3'-carboxy-6'-methoxydiphenylamin-Na-Salz in Form von orangefarbenen Kristallen mit einem Schmelzpunkt oberhalb 250°C erhalten. Daraus wurde durch Hydrierung das 2.4-Diamino-3'-carboxy-6'-methoxydiphenylamin-dihydrochlorid-dihydrat in Form von grauen Kristallen mit einem Schmelzpunkt von 249°C (unter Zersetzung) erhalten.

1.8) <u>2.4-Diamino-2'-carboxy-6'-methyldiphenylamin-trihydrochlorid-dihydrat</u>

Nach dem Verfahren gemäß 1.4 wurde ausgehend von 2-Amino-3-methylbenzoesäure das 2.4-Dinitro-2'-carboxy-6'-methyldiphenylamin-Na-Salz in Form orangefarbener Kristalle mit einem Schmelzpunkt oberhalb 250°C hergestellt. Daraus wurde durch katalytische Hydrierung das 2.4-Diamino-2'-carboxy-6'-methyldiphenylamin-trihydrochlorid-dihydrat in Form von roten Kristallen mit einem Schmelzpunkt von 234°C (unter Zersetzung) erhalten.

1.9) <u>2.4-Diamino-2'-carboxy-3'-methyldiphenylamin-trihydrochlorid-dihydrat</u>

Nach dem Verfahren gemäß 4.1 wurde ausgehend von 2-Amino-6-methylbenzoesäure das 2.4-Dinitro-2'-carboxy-3'-methyldiphenylamin-Na-Salz in Form roter Kristalle mit einem Schmelzpunkt oberhalb 250°C (unter Zersetzung) erhalten. Daraus wurde durch katalytische Hydrierung das 2.4-Diamino-2'-carboxy-3'-methyldiphenylamin-trihydrochlorid-dihydrat in Form eines farblosen Pulvers mit einem Schmelzpunkt von 213°C (unter Zersetzung) erhalten.

...

1.10) 2.4-Diamino-2'-carboxy-5'-chlordiphenylamin-trihydrochlorid-dihydrat

Nach dem Verfahren gemäß 4.1 wurde ausgehend von 2-Amino-4-Chlorbenzoesäure das 2.4-Dinitro-2'-carboxy-5'-chlordiphenylamin-Na-Salz in Form roter Kristalle mit einem Schmelzpunkt oberhalb 250°C (unter Zersetzung) hergestellt. Durch katalytische Hydrierung (in Gegenwart von Raney-Nickel) wurde das 2.4-Diamino-2'-carboxy-5'-chlordiphenylamin-trihydrochlorid-dihydrat in Form eines grauen Pulvers mit einem Schmelzpunkt von 249°C (unter Zersetzung) erhalten.

1.11) 2.4-Diamino-4'-carboxymethyl-diphenylamin-dihydrochlorid

1. Stufe

Eine Mischung aus 7,6 (0,05 Mol) p-Aminophenylessigsäure und 10 g (0,12 Mol) $NaHCO_3$ in 60 ml Ethanol wurden auf 50°C erwärmt, dann wurden 9,3 g (0,05 Mol) 2.4-Dinitrofluorbenzol zugetropft. Nach 3 Stunden sieden unter Rückflußkühlung wurde das Reaktionsprodukt abfiltriert und bei 80°C im Vakuum getrocknet. Es wurden 14,7 g (93 % d.Th.) 2.4-Dinitro-4'-carboxymethyl-diphenylamin-Na-Salz in Form von orangefarbenen Kristallen mit einem Schmelzpunkt von 206°C (unter Zersetzung) erhalten.

2. Stufe

Das 2.4-Dinitro-4'-carboxymethyl-diphenylamin-Na-Salz wurde analog 1.4 (2. Stufe) hydriert. Dabei wurde das 2.4-Diamino-4'-carboxymethyl-diphenylamin-dihydrochlorid in Form von rosa Kristallen mit einem Schmelzpunkt ab 180°C (unter Zersetzung) erhalten.

. . .

2. Anwendungstechnische Prüfungen

Es wurden erfindungsgemäße Haarfärbemittel in Form einer Haarfärbe-Cremeemulsion der folgenden Zusammensetzung hergestellt:

| | | |
|---|---|---|
| Fettalkohol $C_{12-14}$ | | 10 g |
| Fettalkohol $C_{12-14}$ + 2 EO-sulfat, Na-Salz, 28 %ig | | 25 g |
| Wasser | | 60 g |
| Kupplersubstanz | | 0,0075 Mol |
| Entwicklersubstanz | | 0,0075 Mol |
| $Na_2SO_3$ (Inhibitor) | | 1,0 g |
| konzentrierte Ammoniak-Lösung | bis | pH = 9,5 |
| Wasser | ad | 100 g |

Die Bestandteile wurden der Reihe nach miteinander vermischt. Nach Zugabe der Oxidationsfärbemittelvorprodukte und des Inhibitors wurde zunächst mit konzentrierter Ammoniak-Lösung der pH-Wert der Emulsion auf 9,5 eingestellt, dann wurde mit Wasser auf 100 g aufgefüllt.

Die oxidative Entwicklung der Färbung wurde mit 3 %iger Wasserstoffperoxidlösung als Oxidationsmittel durchgeführt. Hierzu wurden 100 g der Emulsion mit 50 g Wasserstoffperoxidlösung (3-%ig) versetzt und vermischt.

Die Färbecreme wurde auf ca. 5 cm lange Strähnen standardisierten, zu 90 % ergrauten, aber nicht besonders vorbehandelten Menschenhaars aufgetragen und dort 30 Minuten bei 27°C belassen. Nach Beendigung des Färbeprozesses wurde das Haar gespült, mit einem üblichen Haarwaschmittel ausgewaschen und anschließend getrocknet.

...

Als Kupplersubstanzen wurden die folgenden Verbindungen eingesetzt:

K 1 = 2.4-Diaminodiphenylamin-4'-sulfonsäure-Na-Salz
K 2 = 2.4-Diaminodiphenylamin-2'-sulfonsäure-Na-Salz
K 3 = 2.4-Diaminodiphenylamin-3'-sulfonsäure-Na-Salz
K 4 = 2.4-Diamino-4'-methyldiphenylamin-2'-sulfonsäure-Na-Salz
K 5 = 2.4-Diamino-4'-carboxy-diphenylamin
K 6 = 2.4-Diamino-3'-carboxy-diphenylamin
K 7 = 2.4-Diamino-2'-carboxy-3'-methyl-diphenylamin
K 8 = 2.4-Diamino-3'-carboxy-6'-methyl-diphenylamin
K 9 = 2.4-Diamino-2'-carboxy-6'-methyldiphenylamin
K 10 = 2.4-Diamino-2'-carboxy-4'-methyldiphenylamin
K 11 = 2.4-Diamino-3'-carboxy-6'-methoxy-diphenylamin
K 12 = 2.4-Diamino-2'-carboxy-5'-chlordiphenylamin

Als Entwicklersubstanzen wurden die folgenden Verbindungen eingesetzt:

E 1 = p-Toluylendiamin
E 2 = N-(2-Hydroxyethyl)-p-phenylendiamin
E 3 = N,N-Bis-(2-hydroxyethyl)-p-phenylendiamin
E 4 = N-Ethyl-N-(2-hydroxyethyl)-p-phenylendiamin
E 5 = N,N-Diethyl-p-phenylendiamin
E 6 = 2-Chlor-p-phenylendiamin
E 7 = N-Benzyl-p-phenylendiamin
E 8 = N-(p-Aminophenyl)-N',N'-bis-(2-hydroxyethyl)-1.3-diaminopropan
E 9 = 2.5-Diaminoanisol
E 10 = 2.5-Diaminopyridin
E 11 = p-Aminophenol
E 12 = 2,4,5,6-Tetraaminopyrimidin
E 13 = 6-Piperidino-2,4,5-triaminopyrimidin

Die mit diesen Oxidationsfarbstoffvorprodukten erhaltenen Haaranfärbungen sind der folgenden Tabelle zu entnehmen:

...

TABELLE

| Anwendungs-beispiel | Kuppler | Entwickler | Nuance der gefärbten Haarsträhne |
|---|---|---|---|
| 2.1 | K 1 | E 1 | dunkelolivbraun |
| 2.2 | K 1 | E 5 | braungrau |
| 2.3 | K 1 | E 11 | rehbraun |
| 2.4 | K 1 | E 12 | olivbraun |
| 2.5 | K 2 | E 1 | dunkelbraun |
| 2.6 | K 2 | E 3 | braungrau |
| 2.7 | K 2 | E 7 | graubraun |
| 2.8 | K 2 | E 11 | rehbraun |
| 2.9 | K 2 | E 12 | olivgrün |
| 2.10 | K 3 | E 1 | dunkelbraun |
| 2.11 | K 4 | E 1 | braun |
| 2.12 | K 5 | E 1 | dunkelbraun |
| 2.13 | K 6 | E 1 | schwarzbraun |
| 2.14 | K 7 | E 1 | dunkelbraun |
| 2.15 | K 8 | E 1 | dunkelbraun |
| 2.16 | K 9 | E 1 | dunkelbraun |
| 2.17 | K 9 | E 4 | rotgrau |
| 2.18 | K 9 | E 12 | dunkelgrün |
| 2.19 | K 9 | E 13 | hellbraun |
| 2.20 | K 10 | E 1 | dunkelbraun |
| 2.21 | K 10 | E 2 | violettgrau |
| 2.22 | K 10 | E 6 | graubraun |
| 2.23 | K 10 | E 9 | purpurgrau |
| 2.24 | K 10 | E 11 | schokoladenbraun |
| 2.25 | K 10 | E 12 | dunkelgrün |
| 2.26 | K 11 | E 1 | braun |
| 2.27 | K 11 | E 2 | blaugrau |
| 2.28 | K 11 | E 8 | violettgrau |
| 2.29 | K 11 | E 10 | rotbraun |
| 2.30 | K 11 | E 12 | olivbraun |
| 2.31 | K 12 | E 1 | schwarzbraun |

...

## Patentansprüche

1. Haarfärbemittel, enthaltend Oxidationsfarbstoffvorprodukte in einem Träger, dadurch gekennzeichnet, daß sie als Oxidationsfarbstoffvorprodukte Aminodiphenylamine der Formel I

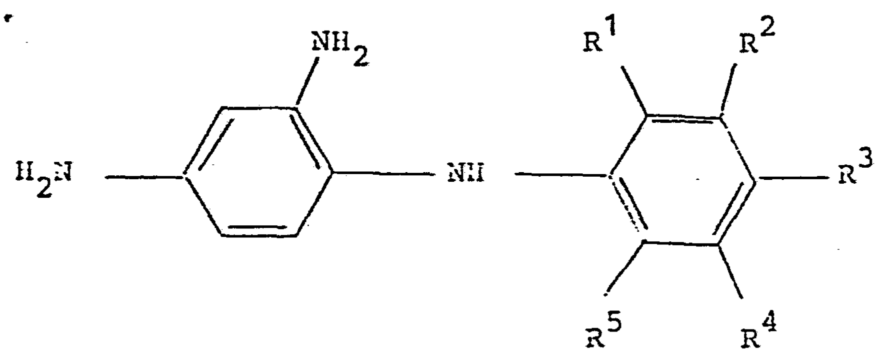

(I)

in der eine der Gruppen $R^1$ - $R^5$ eine $-SO_3H$, -COOH oder eine $-CH_2$-COOH-Gruppe und die übrigen Wasserstoff, eine Alkylgruppe mit 1 - 4 C-Atomen, eine Alkoxygruppe mit 1 - 4 C-Atomen oder ein Chloratom sein können, mit der Maßgabe, daß wenigstens zwei der Gruppen $R^1$ - $R^5$ Wasserstoff darstellen, oder deren Salze als Kupplersubstanzen und die in Oxidationshaarfärbemitteln üblichen Entwicklersubstanzen enthalten.

2. Haarfärbemittel nach Anspruch 1, dadurch gekennzeichnet, daß sie als Kupplersubstanzen Aminodiphenylamine der Formel I nach Anspruch 1, in der eine der Gruppen $R^1$ - $R^5$ eine $-SO_3H$ oder -COOH-Gruppe und die übrigen Wasserstoff oder Methylgruppen sind, wobei wenigstens 3 dieser Gruppen Wasserstoff sind, enthalten.

3. Haarfärbemittel nach Anspruch 1 oder 2, dadurch gekennzeichnet, daß sie als Entwicklersubstanzen aromatische Diamine enthalten.

...

4. Haarfärbemittel nach Anspruch 1 - 3, dadurch gekennzeichnet, daß zusätzlich weitere, bekannte Kupplersubstanzen enthalten sind, daß Entwicklersubstanzen und Kupplersubstanzen im Molverhältnis 1 : 0,5 bis 1 : 2 enthalten sind, daß der Gehalt an Oxidationsfarbstoffvorprodukten 0,2 - 5,0 Gew.-% des Haarfärbemittels und der Gehalt an Aminodiphenylaminen der Formel I 0,05 - 10 Millimol pro 100 g des Haarfärbemittels beträgt.

. . .